# EUROPÄISCHE PATENTANMELDUNG

(11) **EP 0 951 882 A2**
(43) Veröffentlichungstag der Anmeldung: **27.10.1999**
(21) Anmeldenummer: 99107826.2
(22) Anmeldetag: 20.04.1999
(51) Int. Cl.: A61F 9/00, A61B 17/36

(54) **Vorrichtung zur nichtmechanischen Trepanation bei Hornhauttransplantationen**

(30) Priorität: 20.04.1998 DE 19817403
(71) Anmelder: NWL Laser-Technologie GmbH, 91242 Ottensoos (DE)
(72) Erfinder: Langenbucher, Achim, Dr., 90453 Nürnberg (DE); Naumann, Gottfried, Dr., 91054 Erlangen (DE); Seitz, Berthold, Dr., 91056 Erlangen (DE); Küchle, Michael, Prof., 91052 Erlangen (DE); Weimel, Erich, 91242 Ottensoos (DE)
(74) Vertreter: Münich, Wilhelm, Dr.

(57) **Zusammenfassung**

Beschrieben wird eine Vorrichtung zur nichtmechanischen Trepanation bei Hornhauttransplantationen, mit einer Lasereinrichtung sowie einer Strahlführungsoptik, um das von der Lasereinrichtung abgegebene Licht zu einem Operationsbereich zu führen.

Die Erfindung zeichnet sich dadurch aus, daß die Lasereinrichtung einen Festkörperlaser aufweist.

## Beschreibung

Die Erfindung betrifft eine Vorrichtung zur nichtmechanischen Trepanation bei Hornhauttransplantationen.

Die Hornhauttransplantation ist die weltweit am häufigsten durchgeführte Transplantation am menschlichen Körper. So wird die Hornhaut meistens mittels eines Skalpells mechanisch geschnitten und ggflls. lokal abgetragen. Diese mechanische Schnittführung, auch unter dem medizinischen Fachbegriff 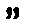 Trepanation" bekannt, erzeugt eine unvermeidliche Gewebedeformation, die zum einen zu einer optischen Verzerrung führen kann und überdies irreversible Gewebeschäden nach sich ziehen kann.

Zur Vermeidung dieser Nachteile ist von der Anmelderin der vorliegenden Patentanmeldung eine Vorrichtung zur nichtmechanischen Trepanation entwickelt worden, die sich eines Excimer-Lasers bedient, der Licht mit einer Wellenlänge von 193 nm emittiert. Mit diesen im Ultraviolettbereich liegendem Licht wird die Hornhaut ohne mechanische Deformation, d.h. mit einem sauberen Schnittbild, ohne Verzerrungen und mit reproduzierbarer Schnittiefe, geschnitten.

Diese Vorrichtung existiert bisher lediglich als Prototyp, da der Excimer-Laser schwierig zu bedienen, äußerst unhandlich, großvolumig und teuer ist. Die Vorteile eines Excimer-Lasers gegenüber anderen bekannten Laser-Systemen betreffen die sehr gute Steuer-und Regelbarkeit von Lichtintensität und Strahlungsenergie. Ferner sind umfangreiche Erfahrungen bei der Materialabtragung mittels Excimer-Laser vorhanden, so daß eine nichtmechanische Trepanation mit der erforderlichen Präzision möglich ist.

Aus obigem ergibt sich, daß eine große Nachfrage nach Systemen besteht, die vergleichsweise einfach handhabbar, kostengünstig, platzsparend sind und dennoch die erforderliche Präzision bei der Trepanation gewährleisten.

Der Erfindung liegt daher die Aufgabe zugrunde, eine einfach handhabbare, kostengünstige, platzsparende Vorrichtung zur nichtmechanischen Trepanation zu schaffen, die zudem die notwendige Präzision gewährleistet.

Die Aufgabe wird durch eine Vorrichtung mit den Merkmalen des Anspruchs 1 gelöst. Vorteilhafte Ausgestaltungen sind in den Unteransprüchen angegeben.

Die erfindungsgemäße Vorrichtung zur nichtmechanischen Trepanation zeichnet sich durch einen Festkörperlaser aus, der vorzugsweise Licht im Infrarotbereich emittiert. Es hat sich überraschenderweise gezeigt, daß die Energie des abgestrahlten Lichtes mit der notwendigen Präzision stabil und in dem gewünschten Bereich auch exakt eingestellt werden kann. Hierbei ist zu Berücksichtigen, daß die Strahlenergie in sehr engen Grenzen gehalten werden muß, da eine zu hohe Strahlenergie thermische Verletzungen der Hornhaut hervorruft und zu geringe Energiemengen, die Hornhaut lediglich beschädigen, jedoch nicht schneiden.

Bei der erfindungsgemäßen Vorrichtung besteht im Gegensatz zu einer mit einem ultraviolettes Licht abstrahlenden Excimerlaser arbeitenden Vorrichtung kein Mutagenitätsrisiko, da Infrarotlicht abgestrahlt wird.

Als besonders vorteilhaft hat sich die Verwendung eines Erbium:YAG-Lasers erwiesen, der z.B. bei einer Wellenlänge von 2.940 nm betrieben wird, da Wasser im Bereich von 2,9 µm eine Absorptionsbande aufweist, so daß die Energie des Laserlichtes sehr effektiv in thermische Energie zum Schneiden der Hornhaut umgewandelt wird.

Der Laserstrahl wird über einen Lichtleiter oder einen Spiegelarm zu einem Operationsmikroskop geführt, an welchem der Operateur die Hornhaut eines Spenderauges ausschneidet.

Die Pulsfrequenz des Festkörperlasers liegt vorzugsweise im Bereich von 2 bis 25 Hz, da hierbei der Energieeintrag auf den erforderlichen Betrag gut einstellbar ist.

Mit einem diffraktiven optischen Element kann das Laserlicht in eine Ringstruktur gewandelt werden, so daß ohne Verwendung einer Maske die Hornhaut in der Form einer Kreisscheibe entlang einer ringförmigen Schnittkante ausgeschnitten wird, wobei die Schnittlinie sehr einfach und exakt alleine durch das diffraktive optische Element eingestellt werden kann.

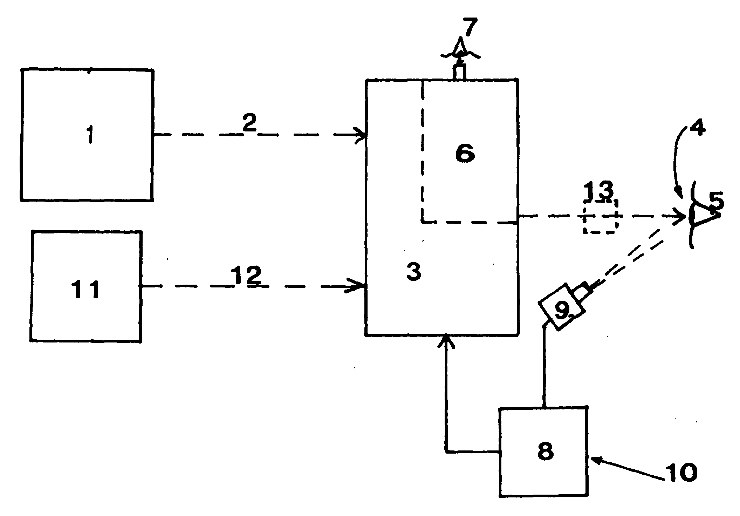

Die Erfindung wird nachstehend ohne Beschränkung des allgemeinen Erfindungsgedankens anhand eines Ausführungsbeispieles unter Bezugnahme auf die einzige Zeichnung exemplarisch beschrieben.

Die erfindungsgemäße Vorrichtung zur nichtmechanischen Trepanation weist eine Lasereinrichtung 1 mit einem Festkörperlaser auf, dessen Lichtstrahl 2 mit einer Strahlführungsoptik 3 zu einem Operationsbereich 4 geführt wird.

Am Operationsbereich 4 wird ein Spenderauge 6 zum Entfernen eines Teils der Hornhaut angeordnet (Trepanation).

Erfindungsgemäß wird ein Festkörperlaser verwendet, der Licht mit einer Wellenlänge, die in einem Absorptionsband von Wasser liegt, emittiert, so daß die Lichtenergie an der Hornhaut in thermische Energie zum Schneiden der Hornhaut umgesetzt wird. Der Festkörperlaser ist vorzugsweise ein Erbium:YAG-Laser, der Licht mit einer Wellenlänge von 2940 nm aussendet. Erfindungsgemäß ist erkannt worden, daß ein relativ einfach handhabbarer Festkörperlaser auch im Bereich von Absorptionsbanden des Wassers Licht mit der erforderlichen Intensität und insbesondere Intensitäts- und Frequenzstabilität aussenden kann.

Die Ausgangsenergie des Lasers ist grundsätzlich regelbar, bspw. im Bereich von 0 bis 1 J, so daß der Energieeintrag an die Hornhautdicke und dgl. angepaßt werden kann. Der Erbium:YAG-Laser wird vorzugsweise mittels einer Blitzlichtlampe angeregt und gepulst betrieben. Die Impulslänge der Laserlichtimpulse wird durch eine an sich bekannte Güteschaltung eingestellt und liegt z.B. im Nanosekundenbereich, wobei die gesamte Lichtleistung und damit der Energieeintrag durch Verändern der Wiederhohlfrequenz im Bereich von 2 bis 25 Hz gesteuert wird. Die Leistung pro Puls wird über eine geschlossene Regelschleife auf einen vorbestimmten Wert gehalten. Diese Steuer- und Regelanordnung erlaubt eine sehr stabile Einstellung der Lichtleistung, wodurch sichergestellt werden kann, daß der Energieeintrag einerseits nicht zu gering ist, wodurch die Hornhaut nur verletzt aber nicht geschnitten werden würde, und andererseits nicht zu groß ist, wodurch die Hornhaut zerstört werden könnte.

Das aktive Material (Erbium:YAG) liegt typischerweise in der Form einer Stange vor, die z.B. eine Länge von 120 mm und einen Durchmesser von 4 mm aufweist.

Anstelle des Erbium:YAG-Laser können auch andere Festkörperlaser eingesetzt werden, sofern sie Licht in einem Absorptionsband von Wasser aussenden, wie z.B. Licht mit einer Wellenlänge von 2,9 µm.

Der Laserstrahl 2 wird von der Strahlführungsoptik 3 zu einem Operationsmikroskop 8 geführt, an welchem der Operateur (Auge 9) den auf das Spenderauge 5 auftreffenden Laserstrahl 2 betrachten kann. Die Strahlführungsoptik 3 weist zur Führung des Laserstrahles 2 z.B. einen Spiegelarm oder eine Faseroptik auf.

Zum Justieren des Laserstrahls bzw. der Strahlführungsoptik 3 ist ein Ziehlstrahllaser 11 parallel zum Arbeitslaser 1 angeordnet, der einen Ziehlstrahl 12 eines sichtbaren, die Hornhaut des Spenderauges 5 nicht beeinträchtigenden Lichtes aussendet. Dieser Ziehlstrahl 12 wird beim Justieren der Strahlführungsoptik 3 in diese eingekoppelt, so daß der Operateur den Strahlweg des sichtbaren Laserstrahls 12 verfolgen und insbesondere seine Position auf dem Spenderauge 5 ohne einer Verletzungsgefahr des Spenderauges bestimmen kann.

Die Strahlführungsoptik ist mit Mittel zum Justieren des Laserstrahls am Spenderauge ausgestattet, die vom Operateur bedient werden können, während er durch das Mikroskop die Position des Laserstrahls 2 auf dem Auge 5 beobachtet. Die Justiermittel sind z.B. X-Y-Galvanometerspiegel, welche den Laserstrahl in einer X-Y-Ebene senkrecht zur Strahlrichtung verschieben können.

Da Bewegungen des Spenderauges 5 nicht vollständig vermieden werden können, ist vorzugsweise ein Eye-Tracking-System 8 vorgesehen, das eine auf das Spenderauge 5 gerichtete Videokamera 9 und eine elektrische Auswerteeinrichtung 10 aufweist. Mit der Videokamera 9 wird die Position des Spenderauges 5 erfaßt, die dann durch die elektrische Auswerteeinrichtung 10 ausgewertet und zum automatischen Nachführen des Laserstrahles 2 an die Strahlführungsoptik 3 weitergeleitet wird. Vorzugsweise ist das Eye-Tracking-System 8 derart in die Strahlführungsoptik 3 und das Mikroskop 6 integriert, daß die Augenbewegungen auch im Mikroskop 6 kompensiert werden, so daß bei einem einmal auf das Spenderauge 5 aufgeschaltetem Eye-Tracking-System 8 der Operateur im Mikroskop ein quasi stillstehendes Spenderauge 5 sieht. Er muß somit den Laserstrahl relativ zum Spenderauge 5 aber nicht bzgl. einer momentanen absoluten Position des Spenderauges 5 einstellen.

Neben der exakten Position des Laserstrahls auf dem Spenderauge 5 muß auch die Kontur des Schnittes eindeutig definiert sein. Zur Festlegung der Schnittkontur sind folgende drei Möglichkeiten vorgesehen:
- Der Laserstrahl 2 wird lediglich grob fokussiert und auf eine auf dem Spenderauge 5 angeordnete Maske (nicht dargestellt) projiziert, wobei ein in der Maske als schmaler Spalt oder als lichtdurchlässiges Material ausgebildetes Schnittmuster abgefahren wird, um die Hornhaut entsprechend dem Verlauf des Schnittmusters zu schneiden.
- Der Laserstrahl 2 wird fein fokussiert und durch die oben beschriebene Scanneroptik mittels der Galvanometerspiegel auf dem Spenderauge 5 entlang der Schnittkontur geführt, so daß ohne Zwischenschaltung einer Maske die Hornhaut geschnitten wird.
- Es wird ein diffraktives optisches Element 13 in den Laserstrahl 2 eingebracht, das den Laserstrahl 2 zu einem Ring aufweitet, wobei der Ring unmittelbar auf das Spenderauge 5 abgebildet wird.

Unabhängig von der Art der angewandten Methode kann zum Justieren der Schnittkontur wiederum der sichtbare Ziehlstrahl 12 des Ziehlstrahllasers 11 verwendet werden.

Bei der Verwendung einer Maske ist vorteilhaft, daß bei einem Abweichen des Laserstrahls 2, bspw. durch eine Fehlfunktion der optischen Strahlführung, das übrige Spenderauge geschützt ist und insbesondere der auszuschneidende Teil der Hornhaut nicht durch den Laserstrahl 2 verletzt werden kann. Da jedoch auch die Maskenoberfläche teilweise von dem Laserstrahl abgetragen wird, ist es zweckmäßig, die hierbei entstehenden Partikel mittels eines Absaugsystems (nicht dargestellt) abzusaugen.

Die beiden anderen Methoden verwenden keine Maske und können deshalb ohne zusätzliches Absaugsystem eingesetzt werden.

Bei Verwendung des optisch diffraktiven Elementes 13 muß der Laserstrahl 2 lediglich einmal exakt gegenüber dem Spenderauge 5 justiert werden, ohne daß der Laserstrahl 2 entlang der Schnittkontur geführt werden braucht. Die Steuerung des Laserstrahls 2 vereinfacht sich deshalb wesentlich im Vergleich zu den beiden anderen Methoden, bei welchen jeweils mit dem Laserstrahl 2 die Schnittkontur abgefahren wird.

Mit der erfindungsgemäßen Vorrichtung zur nichtmechanischen Trepanation, wird erstmals eine handliches, einfach und gefahrlos zu bedienendes Gerät geschaffen, das bei geringen Unterhalts- und Anschaffungskosten einen nichtmechanische Trepanation ertaubt. Dies ermöglicht allen operativen Augenkliniken an den Vorteilen der nichtmechanischen Trepanation - keine Hornhautverkrümmungen durch mechanische Einwirkungen - teilhaben zu können. Dieser Fortschritt bei der Hornhauttransplantation gelang, da die Erfinder der vorliegenden Erfindung erstmals erkannten, daß auch ein Festkörperlaser für eine nichtmechanische Trepanation eingesetzt werden.

### BEZUGSZEICHENLISTE

- 1: Lasereinrichtung
- 2: Laserstrahl
- 3: Strahlführungsoptik
- 4: Operationsbereich
- 5: Spenderauge
- 6: Mikroskop
- 7: Auge des Operateurs
- 8: Eye-Tracking-System
- 9: Videokamera
- 10: Auswerteeinrichtung
- 11: Ziehlstrahllaser
- 12: Ziehlstrahl
- 13: diffraktives optisches Element

## Patentansprüche

1. Vorrichtung zur nichtmechanischen Trepanation bei Hornhauttransplantationen, mit einer Lasereinrichtung (1) sowie einer Strahlführungsoptik (3), um das von der Lasereinrichtung (1) abgegebene Licht zu einem Operationsbereich (4) zu führen,
**dadurch gekennzeichnet,** daß die Lasereinrichtung (1) einen Festkörperlaser aufweist.

2. Vorrichtung nach Anspruch 1,
**dadurch gekennzeichnet,** daß der Festkörperlaser (1) ein Erbium:YAG-Laser ist.

3. Vorrichtung nach Anspruch 1 oder 2,
**dadurch gekennzeichnet,** daß die Lasereinrichtung (1) Infrarotlicht emittiert.

4. Vorrichtung nach einem der Ansprüche 1 bis 3,
**dadurch gekennzeichnet,** daß die Lasereinrichtung (1) Licht mit einer Wellenlänge von 2.940 nm emittiert.

5. Vorrichtung nach einem der Ansprüche 1 bis 4,
**dadurch gekennzeichnet,** daß die Lasereinrichtung (1) gepulst betreibbar ist.

6. Vorrichtung nach Anspruch 5,
**dadurch gekennzeichnet,** daß die Pulsfrequenz im Bereich von 2 bis 25 Hz liegt.

7. Vorrichtung nach Anspruch 5 oder 6,
**dadurch gekennzeichnet,** daß die Lasereinrichtung (1) eine Blitzlichtlampe zur Anregung des aktiven Mediums aufweist.

8. Vorrichtung nach einem der Ansprüche 1 bis 7,
**dadurch gekennzeichnet,** daß das die Lasereinrichtung (1) derart ausgebildet ist, daß die Ausgangsenergie im Bereich von 0 bis 1 Joule regelbar ist.

9. Vorrichtung nach einem der Ansprüche 1 bis 8,
**dadurch gekennzeichnet,** daß in dem Strahlengang von der Lasereinrichtung (1) zum Operationsbereich (8) ein diffraktives optisches Element (13) zur Fokussierung des Laserlichtes (1) in eine Ringstruktur angeordnet ist.

10. Vorrichtung nach einem der Ansprüche 1 bis 9,
**dadurch gekennzeichnet,** daß im Strahlengang von der Lasereinrichtung (1) zum Operationsbereich (8) eine Scanneroptik (3) zum Ablenken des Laserstrahles auf vorbestimmte Punkte bzw. Bahnen im Operationsbereich (8) angeordnet ist.

11. Vorrichtung nach Anspruch 10,
**dadurch gekennzeichnet,** daß die Scanneroptik ein X-Y-Galvanometerspiegelsystem zum Ablenken des Laserstrahles aufweist.

12. Vorrichtung nach Anspruch 10 oder 11,
**dadurch gekennzeichnet,** daß die Scanneroptik einen Ziehlstrahllaser (12) aufweist, der sichtbares, die Hornhaut nicht beeinträchtigendes Licht aussendet, so daß ein Operateur die optische Justierung der Scanneroptik kontrollieren kann.

13. Vorrichtung nach einem der Ansprüche 1 bis 12,
**dadurch gekennzeichnet,** daß die Strahlführungsoptik ein faseroptisches Strahlübertragungssystem aufweist.

14. Vorrichtung nach einem der Ansprüche 1 bis 12,
**dadurch gekennzeichnet,** daß die Strahlführungsoptik einen Spiegelarm aufweist.

15. Vorrichtung nach einem der Ansprüche 1 bis 14,
**dadurch gekennzeichnet,** daß die Strahlführungsoptik ein Eye-Tracking-System (8) aufweist, das die Bewegung des zu operierenden Auges erfaßt und Augenbewegungen durch einen entsprechenden Offset im Strahlengang automatisch ausgleicht.
